# EUROPÄISCHE PATENTSCHRIFT

(11) **EP 2 817 366 B1**
(45) Veröffentlichungstag und Bekanntmachung des Hinweises auf die Patenterteilung: **12.04.2017**
(21) Anmeldenummer: 13705179.3
(22) Anmeldetag: 20.02.2013
(51) Int. Cl.: C08K 5/11

(54) **GEMISCHTE ALKYLBENZYLESTER DER BERNSTEINSÄURE ALS WEICHMACHER**
MIXED ALKYL BENZYL ESTER OF SUCCINIC ACID AS SOFTENER
ESTER D'ALKYLBENZYLE MIXTE D'ACIDE SUCCINIQUE EN TANT QUE PLASTIFIANT

(30) Priorität: 24.02.2012 EP 12156807
(43) Veröffentlichungstag der Anmeldung: 31.12.2014
(73) Patentinhaber: Lanxess Deutschland GmbH, 50569 Köln (DE); BioAmber International S.A.R.L., 1140 Luxembourg (LU)
(72) Erfinder: FACKLAM, Thomas, 51375 Leverkusen (DE)
(74) Vertreter: Michalski Hüttermann & Partner Patentanwälte mbB
(86) Internationale Anmeldenummer: PCT/EP2013/053382
(87) Internationale Veröffentlichungsnummer: WO 2013/124318

(56) Entgegenhaltungen:
- FR-A1- 2 026 170

## Beschreibung

Die vorliegende Erfindung betrifft neue Mischungen von Bernsteinsäureestern auf Basis von Alkyl- und Benzylalkoholen und deren Verwendung als Weichmacher für Kunststoffe.

Zur Verarbeitung von Kunststoffen wie beispielsweise Polyvinylchlorid (PVC) werden seit Jahrzehnten Weichmacher eingesetzt. Weichmacher sind in der Polymerverarbeitung eingesetzte Additive, die die Verarbeitbarkeit, die Flexibilität und die Dehnbarkeit verbessern. Da die Weichmacher nicht fest mit dem Polymeren verbunden sind, können sie migrieren oder sich verflüchtigen. Die zur Erzeugung von Weich-PVC eingesetzten Weichmacher sind überwiegend Phthalsäureester wie die Allzweckprodukte Di-2-ethylhexylphthalat (DEHP), Diisononylphthalat (DINP) und Diisodecylphthalat (DIDP). Zur Verbesserung der Verarbeitungsprozesse in Hinblick auf Geschwindigkeit oder Energieeinsparung können schnell gelierende Weichmacher wie beispielsweise die kurzkettigen Phthalate Dibutylphthalat (DBP), Diisobutylphthalat (DiBP) und Benzylbutylphthalat (BBP) zugesetzt werden.

Die Verwendung von Phthalaten wird aufgrund gesetzlicher Rahmenbedingungen immer weiter eingeschränkt. Ein Beispiel ist das Verbot und die Beschränkung des Einsatzes einiger Phthalate zur der Herstellung von Spielzeug und Babyartikeln (Richtlinie 2005/84/EG des Europäischen Parlaments und des Rates vom 14. Dezember 2005). Auch wurden von der Europäischen Chemikalienagentur (ECHA) mehrere Phthalate in die Liste der Kandidaten der besonders Besorgnis erregenden Stoffe (SVHC) aufgenommen.

Hinzu kommt, dass die Phthalate-Weichmacher auf petrochemisch erzeugten Rohstoffen basieren. Bei deren Herstellung werden Treibhausgase frei gesetzt was in vieler Hinsicht als problematisch anzusehen ist.

Deshalb besteht ein Bedarf an phthalatfreien Weichmachern für Kunststoffe. Weichmacher auf Basis nachwachsender Rohstoffe stehen dabei wegen der Diskussion zu Klima, Nachhaltigkeit und begrenzter Verfügbarkeit fossiler Rohstoffe im Fokus des Interesses. Weichmacher auf Basis von Bernsteinsäure könnten diese Anforderungen erfüllen.

Da neuere Entwicklungen auf dem Gebiet der "grünen" Technologien einen kostengünstigen und effektiven Zugang zu bio-basierter Bernsteinsäure geschaffen haben, ist der Einsatz von bio-basierter Bernsteinsäure zur Herstellung von Weichmachern in technischem Maßstab möglich geworden. In diesem Zusammenhang wurde die Bernsteinsäure als einer von 12 zuckerbasierten Synthesebausteinen von höchstem Wert identifiziert (vgl. z.B. T. Werpy and G. Petersen et al. in Top Value Added Chemicals From Biomass, Volume I: Results of Screening for Potential Candidates from Sugars and Synthesis Gas, Seite 1)

Die Verwendung von Bernsteinsäureestern als Weichmacher für Kunststoffe ist bereits seit längerer Zeit bekannt. So beschreibt z.B. die DE-A 1962500 die Verwendung von Dialkylsuccinaten, insbesondere von Bernsteinsäuredicaprylester, als Weichmacher für fleckenbeständige Folien aus PVC.

Die Verwendung von Estern der Bernsteinsäure als Weichmacher für PVC wird von LeCaptain et al. in Polym. Bull. (2010) 65:589-598 im Aufsatz "Poly(vinyl chloride) plasticized with succinate esters: synthesis and characterization" dargestellt. Dort werden Di-octyl succinat (DOS), Di-hexyl succinate (DHS), Di-butyl succinate (DBS) und Di-ethyl succinate (DES) beschrieben. Durch Infrarot (IR)-, dynamische Differenzkalorimetrie (DSC)-, und dynamisch-mechanische Analyse (DMA) wird die Verträglichkeit der Ester in PVC und deren Potential als Ersatz von Phthalaten qualitativ festgestellt. Für den Verarbeiter wichtige anwendungstechnische Untersuchungen und Aussagen wie Migration und Flüchtigkeit aus dem weichgemachten Polymeren oder dessen Langzeitgebrauchseigenschaften werden nicht gemacht.

Aus EP-A 1 873 198 sind bereits Alkylbenzylester von aromatischen und aliphatischen Polycarbonsäuren bekannt, die als Weichmacher mit niedriger Lösetemperatur für Kunststoffe eingesetzt werden können.

Die genannten, aus dem Stand der Technik bekannten Bernsteinsäureester entsprechen jedoch nicht in allen Punkten den gewünschten Anforderungen an einen guten Weichmacher, insbesondere in Bezug auf Weichmachereffektivität und Verarbeitbarkeit, und sind in dieser Hinsicht noch verbesserungsbedürftig.

Ausgehend vom bekannten Stand der Technik bestand die Aufgabe der vorliegenden Erfindung darin, neue Weichmacher für Kunststoffe auf Basis von Bernsteinsäureestern bereit zu stellen, die über verbesserte Eigenschaften verfügen, insbesondere in Bezug auf die weichmachende Wirkung in Verbindung mit einer verbesserten Verarbeitbarkeit des Endproduktes.

Es wurde nun gefunden, dass Mischungen von Bernsteinsäureestern auf Basis von Alkyl- und Benzylalkoholen besonders gut als Weichmacher für Kunststoffe, insbesondere als Weichmacher für PVC geeignet sind. Überraschenderweise besitzen die erfindungsgemäßen Mischungen nicht nur eine niedrige Lösetemperatur sondern zeichnen sich darüber hinaus auch durch sehr gute weichmachende Eigenschaften aus.

Eine schnellere Verarbeitung der Kunststoffe, oder eine Verarbeitung der Kunststoffe wird bei niedrigeren Temperaturen ermöglicht.

Gegenstand der vorliegenden Erfindung sind Mischungen von Bernsteinsäureestern enthaltend Verbindungen der Formeln

R¹-OC(O)-CH₂-CH₂-C(O)O-R¹ (I)

R¹-OC(O)-CH₂-CH₂-C(O)O-R² (II)

und

R²-OC(O)-CH₂-CH₂-C(O)O-R² (III),

worin
R¹ für einen geradkettigen oder verzweigten Alkylrest steht,
   und
R² für einen gegebenenfalls substituierten Benzylrest steht
dadurch gekennzeichnet, dass die Menge an Verbindung der Formel (I) 10 bis 50 Gew%, die Menge an Verbindung der Formel (II) 25 bis 75 Gew.-% und die Menge an Verbindung der Formel (III) 10 bis 50 Gew.-%, bezogen auf 100 Prozent der Mischung, beträgt.

Bevorzugt sind Mischungen von Bernsteinsäureestern wobei in den Formeln (I), (II) und (III) R¹ für einen geradkettigen oder verzweigten Alkylrest mit 1 bis 12 Kohlenstoffatomen steht, und R² für einen gegebenenfalls durch C₁- bis C₄-Alkyl oder Halogen substituierten Benzylrest steht.

Besonders bevorzugt sind Mischungen von Bernsteinsäureestern wobei in den Formeln (I), (II) und (III)
R¹ für einen geradkettigen oder verzweigten Alkylrest mit 8 bis 12 Kohlenstoffatomen steht, und R² für einen gegebenenfalls durch C₁- bis C₄-Alkyl substituierten Benzylrest steht.

Ganz besonders bevorzugt sind Mischungen von Bernsteinsäureestern wobei in den Formeln (I), (II) und (III)
R¹ für einen geradkettigen oder verzweigten Alkylrest mit 8, 9, 10 oder 11 Kohlenstoffatomen steht, und
R² für Benzyl (= -CH₂-C₆H₅) steht.

Als geradkettige oder verzweigte Alkylreste in der Bedeutung von R¹ kommen beispielsweise in Frage: Methyl-; Ethyl-; Propyl- wie n-Propyl-, iso-Propyl-; Butyl- wie n-Butyl-, sek.-Butyl-, Isobutyl-; Amyl-; Hexyl-, wie n-Hexyl-, 1,4-Dimethylbutyl-; n-Heptyl-; Octyl- wie Isooctyl-, n-Octyl-, 2-Ethylhexyl-; Nonyl- wie n-Nonyl- und Isononyl-; Decyl-. wie n-Decyl-, Isodecyl-; und Dodecyl- wie n-Dodecyl- und Isododecylreste und deren gesamte verschiedene isomere Formen.

Als gegebenenfalls substituierte Benzylreste in der Bedeutung von R² kommen beispielsweise in Frage: Methylbenzyl-, Dimethylbenzyl-, Trimethylbenzyl-, Ethylbenzyl-, Isopropylbenzyl- und tert. Butylbenzyl-, Chlorbenzyl-, Dichlorbenzyl-, Trichlorbenzyl-, Brombenzyl-, oder Dibrombenzylreste

Insbesondere bevorzugt sind Mischungen von Bernsteinsäurealkylestern worin in den Formeln (I), (II) und (III) R¹ für 2-Ethylhexyl, n-Octyl, Isooctyl-, n-Nonyl-, Isononyl-; 3,5,5-Trimethylhexyl, n-Decyl-, Isodecyl-; n-Dodecyl- oder Isododecyl steht und R² für Benzyl steht.

In den erfindungsgemäßen Mischungen beträgt die Menge an Verbindung der Formel (I) bevorzugt 15 bis 35 Gew.-% und ganz besonders bevorzugt 20 bis 30 Gew.-%, die Menge an Verbindung der Formel (II) bevorzugt 35 bis 65 Gew.-% und ganz besonders bevorzugt 40 bis 60 Gew.-%, und die Menge an Verbindung der Formel (III) bevorzugt 15 bis 35 Gew.-% und ganz besonders bevorzugt 20 bis 30 Gew.-% jeweils bezogen auf 100 Prozent der Mischung.

Ebenfalls besonders bevorzugt sind b) Mischungen von Bernsteinsäurealkylestern die dadurch gekennzeichnet sind, dass sie die Verbindungen der Formeln (I) und (III) enthalten. In den Mischungen b) haben die Reste R¹ und R² jeweils die oben angegebenen allgemeinen und
bevorzugten Bedeutungen. Von den Mischungen b) sind wiederum solche ganz besonders bevorzugt, worin in den Formeln (I) und (III) R¹ für 2-Ethylhexyl, n-Octyl, Isooctyl-, n-Nonyl-, Isononyl-; 3,5,5-Trimethylhexyl, n-Decyl-, Isodecyl-; n-Dodecyl- oder Isododecyl steht und R² für Benzyl steht.

In den Mischungen b) beträgt die Menge an Verbindung der Formel (I) 15 bis 95 Gew.-%, bevorzugt 25 bis 75 Gew.-% und ganz besonders bevorzugt 40 bis 60 Gew.-% und die Menge an Verbindung der Formel (III) 85 bis 5 Gew.-%, bevorzugt 75 bis 25 Gew.-% und ganz besonders bevorzugt 60 bis 40 Gew.%, jeweils bezogen auf 100 Prozent der Mischung.

Die Verbindungen der Formel (II), worin R¹ für Isononyl-, 3,5,5-Trimethylhexyl-, n-Decyl-, Isodecyl-, Isoundecyl oder n-Dodecyl- steht, und R² für Benzyl steht, sind neu und ebenfalls Gegenstand der vorliegenden Erfindung. Die Verbindungen (II) eignen sich hervorragend als Weichmacher für Kunststoffe.

Die erfindungsgemäßen Mischungen können nach unterschiedlichen Verfahren hergestellt werden. So ist es beispielweise möglich zwei unterschiedliche monofunktionelle Alkohole der Formeln R¹-OH (IV) und R²-OH (V), worin R¹ und R² die oben, für die Formeln (I) bis (III) angegebenen, allgemeinen und bevorzugten Bedeutungen haben, mit Bernsteinsäure, in an sich bekannter Weise, unter Abspaltung von Wasser umzusetzen. Das Verfahren kann in einem oder in zwei Schritten durchgeführt werden. Bei der Durchführung in einem Schritt werden alle Reaktionsteilnehmer im Wesentlichen gleichzeitig miteinander in Kontakt gebracht und umgesetzt. Bei der Umsetzung in zwei Schritten werden in einem ersten Schritt die Bernsteinsäure oder ein Derivat davon mit einem Alkohol umgesetzt und die dabei erhaltene Reaktionsmischung mit dem zweiten Alkohol umgesetzt. Die Reaktionsmischung kann mit einem Lösemittel, das auch als Schleppmittel zur Ausschleusung von Reaktionswasser dienen kann, verdünnt werden. Die zur Bildung des Esters eingesetzten monofunktionellen Alkohole können gleichzeitig als Schleppmittel und im Überschuss eingesetzt werden. Die Veresterung der Bernsteinsäure kann mit oder ohne typische, dem Fachmann geläufige Katalysatoren durchgeführt werden.

Die Herstellung ist wie beispielsweise durch Mischung der Einzelkomponenten (I), (II) und (III) in unterschiedlichen Mengen, möglich.

Den oben beschriebenen Umsetzungen können sich dem Fachmann geläufige Reinigungsoperationen, wie zum Beispiel Extraktion, insbesondere wässrige Wäsche, Destillation, Wasserdampfdestillation, Adsorption und / oder Filtration anschließen.

Weiterer Gegenstand der vorliegenden Erfindung ist ein Verfahren zur Herstellung einer erfindungsgemäßen Estermischung enthaltend die Verbindungen der Formeln

R¹-OC(O)-CH₂-CH₂-C(O)O-R¹ (I),

R¹-OC(O)-CH₂-CH₂-C(O)O-R² (II)

und

R²-OC(O)-CH₂-CH₂-C(O)O-R² (III),

worin
R¹ für einen geradkettigen oder verzweigten Alkylrest steht, und
R² für einen gegebenenfalls substituierten Benzylrest steht,
das dadurch gekennzeichnet ist, dass man in einem Verfahrensschritt oder in zwei aufeinander folgenden Verfahrensschritten zwei unterschiedliche monofunktionelle Alkohole der Formeln R¹-OH (IV) und R²-OH (V), worin R¹ und R² die oben, für die Formeln (I) bis (III) angegebenen, allgemeinen und bevorzugten Bedeutungen haben, bei einer Temperatur von 50 bis 250°C und gegebenenfalls bei einem Druck von 2 mbar bis 4 bar und gegebenenfalls in Gegenwart eines Katalysators mit Bernsteinsäure umsetzt und das gebildete Reaktionswasser durch geeignete Maßnahmen, beispielsweise Destillation, aus der Mischung entfernt.

Als Alkohole der Formel (IV) kommen beispielsweise in Frage: Methyl-; Ethyl-; Propyl- wie n-Propyl-, iso-Propyl-; Butyl- wie n-Butyl-, sek.-Butyl-, Isobutyl-; Amyl-; Hexyl-, wie n-Hexyl-, 1,4-Dimethylbutyl-; n-Heptyl-; Octyl- wie Isooctyl-, n-Octyl-, 2-Ethylhexyl-; Nonyl- wie n-Nonyl- und Isononyl-; Decyl-. wie n-Decyl-, Isodecyl-; und Dodecyl- wie n-Dodecyl- und Isododecylalkohol und deren gesamte verschiedene isomere Formen.

Als Alkohole der Formel (V) kommen beispielsweise in Frage: Benzylalkohol sowie Alkylsubstituierte Benzylalkohole wie Methylbenzylalkohol, Ethylbenzylalkohol, Dimethylbenzylalkohol, Trimethylbenzylalkohol, Isopropylbenzylalkohol, tert. Butylbenzylalkohol, weiterhin Halogensubstituierte Benzylalkohole wie Chlorbenzylalkohol, Dichlorbenzylalkohol, Trichlorbenzylalkohol, Brombenzylalkohol, Dibrombenzylalkohol und ähnliche.

Als Katalysatoren eignen sich prinzipiell Verbindungen der Formel MXn worin M für ein Metallkation steht, ausgewählt aus der Gruppe der Metalle Titan, Zirkonium, Vanadium, Aluminium, Eisen, Zinn, und X für ein Anion ausgewählt aus der Gruppe, -CO₃²⁻, Cl⁻, Br⁻, I⁻; -OR⁻ mit R ausgewählt aus Methyl, Ethyl, n-Propyl, i-Propyl, n-Butyl, i-Butyl, t-Butyl; Carboxylate insbesondere Hexanoat, Heptanoat, Octanoat, 2-Ethylhexanoat, Stearat, Palmitat, Oxalat; und worin n für die Oxidationszahl des Metalls, bevorzugt für 2, 3 oder 4 steht. Alternativ können auch starke Brönstedsäuren wie Schwefelsäure, saure Sulfate, wie z.B. Methylsulfat, Ethylsulfat, Propylsulfat, Butylsulfat, Hexylsulfat aber auch KHSO₄ bzw. NaHSO₄ aromatische Sulfonsäuren insbesondere para-Toluolsulfonsäure, Benzolsulfonsäure erfolgreich eingesetzt werden.

Ebenfalls Gegenstand der vorliegenden Erfindung ist ein Verfahren zur Herstellung einer erfindungsgemäßen Estermischung enthaltend die Verbindungen der Formeln

R¹-OC(O)-CH₂-CH₂-C(O)O-R¹ (I)

und

R²-OC(O)-CH₂-CH₂-C(O)O-R² (III),

worin
R¹ für einen geradkettigen oder verzweigten Alkylrest steht, und
R² für einen gegebenenfalls substituierten Benzylrest steht,
das dadurch gekennzeichnet ist, dass man die einzelnen Verbindungen der Formeln (I) und (II) miteinander vermischt.

Ebenfalls Gegenstand der vorliegenden Erfindung ist ein Verfahren zur Herstellung von Verbindungen der Formel

R¹-OC(O)-CH₂-CH₂-C(O)O-R² (II)

worin
R¹ für Isononyl-, 3,5,5-Trimethylhexyl-, n-Decyl-, Isodecyl-, Isoundecyl oder n-Dodecyl- steht, und
R² für einen Benzylrest steht,
das dadurch gekennzeichnet ist, dass man die Verbindung der Formel (II) aus dem Estergemisch der Formeln (I), (II), (III) trennt, oder in einem Zweistufenprozess Säure bzw. Säureanhydrid mit den entsprechenden Alkoholen der Formeln (IV) und (V) oder dessen oder Syntheseäquivalenten nacheinander zur Reaktion bringt, verbunden mit einem möglichen Reinigungsschritt nach Stufe 1 oder 2.

Falls die zur Herstellung der erfindungsgemäßen Bernsteinsäureester Mischungen eingesetzte Bernsteinsäure aus bio-basierten Rohstoffen hergestellt wurde, z.B. durch einen mikrobiologischen Fermentationsprozess, so können Verunreinigungen in der Bernsteinsäure enthalten sein, die sich auch in den erfindungsgemäßen Mischungen wiederfinden können. Typische Verunreinigungen, die durch die eingesetzten Mikroorganismen in die Endprodukte gelangen können sind Stickstoff- und Schwefel-haltige Verbindungen.

Bevorzugt enthalten die erfindungsgemäßen Estermischungen weniger als 1000 ppm Massenanteil an Stickstoffatomen und weniger als 50 ppm Massenanteil an Schwefelatomen jeweils bezogen auf die Mischung. Besonders bevorzugt sind erfindungsgemäße Mischungen die 0,01 bis 750 ppm Massenanteil an Stickstoffatomen und 0,0001 bis 40 ppm Massenanteil an Schwefelatomen jeweils bezogen auf die Mischung enthalten.

Ebenfalls Gegenstand der vorliegenden Erfindung sind Estermischung enthaltend Bernsteinsäurealkylester der Formeln (I), (II) und (III) das dadurch gekennzeichnet ist, dass die Bernsteinsäurealkylester der Formeln (I), (II) und (III) von Biomasseressourcen stammen und in der Mischung 0,01 ppm bis 1000 ppm Massenanteil an Stickstoffatomen und 0,01 ppm bis 50 ppm Massenanteil an Schwefelatomen jeweils bezogen auf die Mischung enthalten ist.

Diese erfindungsgemäßen Estermischungen können hergestellt werden, indem man bei der Durchführung des erfindungsgemäßen Herstellungsverfahrens Alkohole der Formeln (IV) und (V) verwendet.

Die neuen Bernsteinsäureester Mischungen eignen sich hervorragend als Weichmacher für Kunststoffe.

Weiterer Gegenstand der Erfindung ist die Verwendung einer erfindungsgemäßen Bernsteinsäureester Mischung als Weichmacher für Kunststoffe.

Als Kunststoffe eignen sich z.B Polyvinylchlorid (PVC), Vinylchlorid-basierte Copolymere, Polyvinylidenchlorid, Polyvinylacetale, Polyvinylbutyral, Polyacrylate, Polymethacrylate, Polyalkylmethacrylate wie z.B. Poly(methylmethacrylat), Polyamide, Polyurethane, Polylactide, Polymilchsäuren, Polyvinylacetat, Cellulose und seine Derivate, Ethylenvinylacetate, Kautschukpolymere wie Acrylnitril-Butadien-Kautschuk, hydrierter Acrylnitril-Butadien-Kautschuk, Styrol-Butadien-Kautschuk, Chloroprenkautschuk, Ethylen-Propylen-Kautschuk, Acrylatkautschuk und Naturkautschuk. Bevorzugt werden die erfindungsgemäßen Bernsteinsäureester Mischungen als Weichmacher und Verarbeitungshilfen für PVC und Polyacrylate eingesetzt..

Die mit den neuen Weichmachern hergestellten Kunststoffe zeichnen sich durch eine geringe Härte und hohe Flexibilität im Vergleich zu den aus dem Stand der Technik bekannten mit Weichmachern auf Basis von einkomponentigen Bernsteinsäureestern hergestellten Kunststoffen aus.

Durch den Einsatz der erfindungsgemäßen Weichmacher kann zur Erzielung der gleichen Härte in den Endprodukten die Menge an Weichmacher kostensparend reduziert werden im Vergleich zum Einsatz der bekannten und weniger effizienten, symetrischen Bernsteinsäureester. Auch bedeutet die Einsparung von Weichmachern bzw. die Reduktion der Menge an eingesetztem Weichmacher bessere Endprodukteigenschaften in Hinblick auf Flüchtigkeit und Migration des Weichmachers.

Gegenstand der Erfindung ist auch die Verwendung der erfindungsgemäßen Bernsteinsäureester Mischungen als Verarbeitungshilfen und Weichmacher in Klebstoffen, Klebstoffkomponenten, Klebdichtstoffen, Klebdichtstoffkomponenten, Dichtungsmassen, Dichtungsmassenkomponenten, oder Beschichtungsmassen, in Farben, Tinten oder Lacken, in Plastisolen inklusive PVC-Plastisolen, und als Weichmacher in Kunststoffen oder Kunststoffkomponenten, bevorzugt in Polyvinylchlorid.

Weiterer Gegenstand der vorliegenden Erfindung sind Weichmacherzubereitungen enthaltend eine erfindungsgemäße Bernsteinsäureester Mischung sowie gegebenenfalls weitere übliche Zusatzstoffe. Als solche Zusatzstoffe kommen beispielsweise weitere Weichmacher, Stabilisatoren, Antioxidantien, Gleitmittel, Füllstoffe, Pigmente, Flammschutzmittel, Lichtstabilisatoren, Treibmittel, Kicker, polymere Verarbeitungshilfsmittel, Schlagzähverbesserer, optische Aufheller, Antistatika oder Biostabilisatoren in Frage.

Als PVC-Typen kommen Suspensions-, Masse-, Mikrosuspensions- oder Emulsions-PVC in Frage. Dabei können die Gemische alleine oder in Kombination mit anderen Weichmachern eingesetzt werden. Die erfindungsgemäßen Weichmacher werden im allgemeinen zu 10 bis 200 Teilen, bevorzugt zu 20 bis 150 Teilen, jeweils auf 100 Teile Kunststoff eingesetzt.

Die neuen Weichmacherzubereitungen ermöglichen die Herstellung von Endprodukten mit guten Tieftemperatureigenschaften. Bevorzugt werden die erfindungsgemäßen Gemische zur Herstellung von Plastisolen, bevorzugt von Plastisolen auf Basis von PVC, eingesetzt. Eingesetzt in PVC Plastisolen erlauben die niedrigviskosen Bernsteinsäureestermischungen die Herstellung von niedrigviskosen, lagerstabilen Plastisolen.

Die unter Verwendung der erfindungsgemäßen Weichmacherzubereitungen hergestellten Kunststoffe, insbesondere das Polyvinylchlorid, können neben den erfindungsgemäßen Weichmacherzubereitungen auch noch andere, geeignete Hilfs- und Zusatzstoffe enthalten. Beispiele hierfür sind weitere Weichmacher, Stabilisatoren, Antioxidantien, Gleitmittel, Füllstoffe, Pigmente, Flammschutzmittel, Lichtstabilisatoren, Treibmittel, Kicker, polymere Verarbeitungshilfsmittel, Schlagzähverbesserer, optische Aufheller, Antistatika oder Biostabilisatoren.

Die vorliegende Erfindung betrifft weiterhin ein Verfahren zu Herstellung von weichgemachten Kunststoffen, insbesondere von weichgemachtem PVC, das dadurch gekennzeichnet ist, dass man in einem ersten Schritt PVC, insbesondere Emulsions- und Microsuspensions-PVC, bei 10 bis 60 °C mit der erfindungsgemäßen Weichmacherzubereitung und ggf. weiteren Hilf- und Zusatzstoffen vermischt, wobei auf 100 Teile Kunststoff 10 bis 200 Teile des erfindungsgemäßen Weichmachers eingesetzt werden. In einem zweiten Schritte wird dieses Plastisol in Form gebracht und bei Temperaturen von 140 bis 200°C zum Endartikel verarbeitet.

### Herstellungsbeispiele und technische Prüfung

Die folgenden Beispiele dienen der Erläuterung der vorliegenden Erfindung und sollen in keiner Weise als Einschränkung verstanden werden.

### Herstellung einer erfindungsgemäßen Bernsteinsäureester Mischung

### Beispiel 1:

In einem mit Stickstoffgas inertisiertem 1 1 Mehrhalsrundkolben mit Wasserabscheider und Rückflusskühler, Innenthermometer und Rührer wurden 177 g Bernsteinsäure , 268 g 2-Ethyl-1-hexanol und 221 g Benzylalkohol in 175 ml Xylol vorgelegt. Nach Zugabe von 0,3 g Tetrabutyltitanat wurde der Ansatz unter Rühren zum Rückfluß erwärmt und gebildetes Reaktionswasser ausgekreist. Der Reaktionsverlauf wurde mittels Titration [Säurezahl] und Wasserabscheidung verfolgt. Nach Erreichen einer Säurezahl kleiner gleich 1 wurde die Reaktion beendet. Nach Umbau der Apparatur wurde Xylol und der Überschuß an Alkohol im Vakuum, beginnend bei 20 mbar, abdestilliert; die Sumpftemperatur betrug zum Ende der Destillation 190°C. Nach Abkühlen wurde der Katalysator mit Wasser und wässriger Natriumcarbonatlösung gewaschen. Anschließend wurden beginnend bei 120°C und 20 mbar die flüchtigen Bestandteile aus der organischen Phase destillativ entfernt. und die Mischung anwendungstechnisch als Weichmacher geprüft. Die Ausbeute betrug 435 g (91%). Die Zusammensetzung nach GC Flächenprozenten ist: 29,8% Di-(2-ethylhexyl)succinat, 49,7% Benzyl-2-ethylhexylsuccinat und 20,3% Dibenzylsuccinat.

### Beispiele 2-7:

In Analogie zu Beispiel 1 wurden weitere erfindungsgemäße Estermischungen durch Umsetzung von Bernsteinsäure mit den entsprechenden Alkyl- und Benzylalkoholen hergestellt. Die Ergebnisse der anwendungstechnischen Prüfung für die Beispiele 1 bis 7 sind in Tabelle 1 dargestellt.

### Anwendungstechnische Prüfung

### Bestimmung der Lösetemperatur:

48,0 g der zu prüfenden Substanz wurden in ein Becherglas mit Magnetrührstab und Thermometer eingewogen. Das Becherglas wurde auf einen Magnetheizrührer in eine Halterung zwischen eine Lampe und eine Photozelle gesetzt. Über eine Photozelle wurde die Änderung in der Lichtdurchlässigkeit der Probe registriert. Dann wurden 2 g Polyvinylchlorid (Vinnolit^{®} S4170; Vinnolit GmbH & Co. KG, Deutschland) und mittels Pipette 2 Tropfen PVC-Stabilisator (Organozinnstabilisator) zugesetzt. Das Polyvinylchlorid wurde in den Weichmacher eingerührt und unter Rühren zügig mit 5-8°C pro Minute auf 100°C aufgeheizt und anschließend mit durchschnittlich 3°C pro Minute weiter aufgeheizt. Die Lösetemperatur galt als erreicht, wenn 3 Minuten hintereinander keinerlei Anstieg des Durchlässigkeitswertes durch die Photozelle mehr registriert wurde und das Polyvinylchlorid gelöst war. Bei Erreichen der Temperatur des Becherglasinhaltes von 200°C wurde die Messung abgebrochen. Eine niedrige Lösetemperatur zeigt eine gute Verträglichkeit des Weichmachers und der Weichmacherzubereitungen mit Polyvinylchlorid und ist ein Indikator für eine schnelle Verarbeitbarkeit.

### Bestimmung der Härte:

Zur Bestimmung der Härte wurde eine weichmacherhaltige PVC-Pulvermischung auf einem Zweiwalzenstuhl homogenisiert und geliert; anschließend wurde der Compound zu Prüfplatten gepresst und die Härte mit einem Zwick Shorehärtegerät bestimmt. Die Härte (Shore A) wurde an glatten und ebenen Prüfkörpern der Dimension 6 mm x 40 mm x 50 mm gemessen. Niedrige Shore A Härten bedeuten weichere Produkte und sind ein Maß für die Effizienz der Weichmacher.

In einer Porzellanschale wurden 100 g Polyvinylchlorid (Vinnolit^{®} S4170, Vinnolit GmbH & Co. KG, Deutschland), mit 60 phr (parts per hundred resin) Weichmacher bzw. Weichmacherzubereitung und 3 phr PVC-Stabilisator (Ca/Zn-Carboxylat) mit einem Stab so gemischt, dass die flüssigen Bestandteile vom Pulver gut aufgenommen wurden und nicht an dem Gefäß haften. Die so erhaltene Pulvermischung wurde portionsweise in den Walzenspalt (0,7 mm) eines Zweiwalzenstuhles bei 165°C Walzentemperatur gegeben und homogenisiert und geliert. Nach der Fellbildung wurde der Walzenspalt auf 1 mm erweitert. Das Mischergebnis wurde durch häufiges Einschlagen des Walzfelles verbessert. Nach einer Misch- und Verarbeitungszeit von 10 Minuten wurde das Walzfell abgenommen. Nach Portionierung wurden Prüfkörper der Dimension 6 mm x 40 mm X 50 mm gepresst. Die Temperatur der Presse betrug 170 °C; die Presszeit betrug insgesamt 10 Minuten, davon 7 Minuten Aufheizphase mit einem Druck < 10 bar und 3 Minuten Presszeit unter Hochdruck > 100 bar. Nach Abkühlen unter Druck in einer Kühlpresse auf maximal 30°C wurden die Prüfkörper entformt. Von den Prüfkörpern wurde nach frühestens 24 Stunden Lagerung bei 23 °C die Shore A Härte mit einem Zwick Typ H04.3150 nach DIN 53505 an fünf verschiedenen Stellen unter Notierung des Mittelwertes bestimmt.

**Tabelle 1:**

| | | | |
|---|---|---|---|
| Für die erfindungsgemäßen Estermischung der Beispiele 1 bis 7 und das nicht erfindungsgemäße Beispiel 7a wurde die Shore A Härte von Prüfköpern mit einem Gehalt von jeweils 60 phr an Weichmacher bestimmt. Eine niedriger Shore A Härtewert ist eine Aussage über die gute weichmachende Eigenschaft (Effizienz) des Weichmachers. Weiterhin wurden die Lösetemperaturen bestimmt. Die Lösetemperatur dient als Nachweis der Verträglichkeit eines Weichmachers mit den jeweiligen Kunststoffen oder Polymeren. Eine niedrige Lösetemperatur zeigt in diesem Fall eine gute Verträglichkeit des Weichmachers mit Polyvinylchlorid und eine schnelle Verarbeitbarkeit an. | | | |

| | **Beispiele** | **Shore A Härte** | **Lösetemperatur** |
|---|---|---|---|
| 1 | Mischung Benzyl-2-ethylhexylsuccinat | 70 | 134°C |
| 2 | Mischung Benzyl-isononylsuccinat | 71 | 140°C |
| 3 | Mischung Benzyl-3,5,5-trimethylhexylsuccinat | 74 | 147°C |
| 4 | Mischung Benzyl-1-decylsuccinat | 73 | 149°C |
| 5 | Mischung Benzyl-isodecylsuccinat | 72 | 146 |
| 6 | Mischung Benzyl-isoundecylsuccinat | 75 | 155 |
| 7 | Mischung Benzyl-1-dodecylsuccinat | 93 | 165 |
| 7a | Di-1-dodecylsuccinat | Keine Weichmachung | 196 |

Aus Tabelle 1 geht hervor, dass die erfindungsgemäßen Estermischungen über gute weichmachende Eigenschaften in Verbindung mit einer niedrigen Lösetemperatur verfügen. Damit können die erfindungsgemäßen Estermischungen als effiziente Weichmacher eingesetzt werden, die darüber hinaus auch eine Verarbeitung des PVC bei niedrigen Temperaturen erlauben was wiederum zu Energieeinsparungen und kürzeren Zeiten bei den Herstellzyklen führt. Somit ermöglicht der Einsatz der erfindungsgemäßen Weichmachermischungen überraschenderweise weichere Produkte oder eine kosteneffiziente Reduktion des Weichmacheranteils zur Erzielung von Produkten mit gleicher Härte. Ein geringerer Einsatz der potentiell migrierenden oder flüchtigen Weichmacher erlaubt die Herstellung von Endprodukten mit besseren Eigenschaften in Hinblick auf Flüchtigkeit und Migration des Weichmachers. Der Vergleich von Beispiel 7 und 7a zeigt den positiven Effekt des Benzylrestes in der erfindungsgemäßen Mischung Benzyl-1-dodecylsuccinat auf Härte und Lösetemperatur gegen über dem Di-1-dodecylsuccinat.

## Patentansprüche

1. Mischung von Bernsteinsäureestern enthaltend Verbindungen der Formeln
R¹-OC(O)-CH₂-CH₂-C(O)O-R¹ (I)
R¹-OC(O)-CH₂-CH₂-C(O)O-R² (II),
und
R²-OC(O)-CH₂-CH₂-C(O)O-R² (III),
worin
R¹ für einen geradkettigen oder verzweigten Alkylrest steht, und
R² für einen gegebenenfalls substituierten Benzylrest steht,
**dadurch gekennzeichnet, dass** die Menge an Verbindung der Formel (I) 10 bis 50 Gew.%, die Menge an Verbindung der Formel (II) 25 bis 75 Gew.-% und die Menge an Verbindung der Formel (III) 10 bis 50 Gew.-%, bezogen auf 100 Prozent der Mischung, beträgt.

2. Estermischung gemäß Anspruch 1, wobei in den Formeln (I), (II) und (III) R¹ für einen geradkettigen oder verzweigten Alkylrest mit 1 bis 12 Kohlenstoffatomen steht, und R² für einen gegebenenfalls durch C₁- bis C₄-Alkyl oder Halogen substituierten Benzylrest steht.

3. Estermischung gemäß Ansprüchen 1 und 2, **dadurch gekennzeichnet, dass** in den Formeln (I), (II) und (III) R¹ für einen geradkettigen oder verzweigten Alkylrest mit 8 bis 12 Kohlenstoffatomen steht, und R² für einen Benzylrest steht.

4. Estermischung gemäß wenigstens einem der Ansprüche 1 bis 3, **dadurch gekennzeichnet, dass** die Menge an Verbindung der Formel (I) 15 bis 35 Gew.-% und ganz besonders bevorzugt 20 bis 30 Gew.-%, die Menge an Verbindung der Formel (II) 35 bis 65 Gew.-% und ganz besonders bevorzugt 40 bis 60 Gew.-%, und die Menge an Verbindung der Formel (III) 15 bis 35 Gew.-% und ganz besonders bevorzugt 20 bis 30 Gew.-%, jeweils bezogen auf 100 Prozent der Mischung, beträgt.

5. Mischung von Bernsteinsäureestern enthaltend Verbindungen der Formeln
R¹-OC(O)-CH₂-CH₂-C(O)O-R¹ (I)
und
R²-OC(O)-CH₂-CH₂-C(O)O-R² (III),
wobei die Reste R¹ und R² die in den Ansprüchen 1 bis 3 angegebenen allgemeinen und bevorzugten Bedeutungen haben, **dadurch gekennzeichnet, dass** die Menge an Verbindung der Formel (I) 15 bis 95 Gew.-% und die Menge an Verbindung der Formel (III) 85 bis 5 Gew.-%, bezogen auf 100 Prozent der Mischung, beträgt.

6. Estermischung gemäß Anspruch 5, **dadurch gekennzeichnet, dass** die Menge an Verbindung der Formel (I) 25 bis 75 Gew.-% und ganz besonders bevorzugt 40 bis 60 Gew.-%, und die Menge an Verbindung der Formel (III) 75 bis 25 Gew.-% und ganz besonders bevorzugt 60 bis 40 Gew.-%, jeweils bezogen auf 100 Prozent der Mischung, beträgt.

7. Bernsteinsäurealkylester der Formel
R¹-OC(O)-CH₂CH₂C(O)O-R² (II)
worin
R¹ für Isononyl, 3,5,5-Trimethylhexyl, 1-Decyl, Isodecyl, Isoundecyl oder n-Dodecyl steht, und
R² für Benzyl steht.

8. Estermischung gemäß wenigstens einem der Ansprüche 1 bis 6, **dadurch gekennzeichnet, dass** die Bernsteinsäureester der Formeln (I), (II) und (III) von Biomasseressourcen stammen und in der Mischung 0,01 ppm bis 1000 ppm Massenanteil) an Stickstoffatomen und 0,01 ppm bis 50 ppm Massenanteil an Schwefelatomen jeweils bezogen auf die Mischung enthalten ist.

9. Verfahren zur Herstellung einer Estermischung gemäß Anspruch 1, **dadurch gekennzeichnet ist, dass** man in einem Verfahrensschritt oder in zwei aufeinander folgenden Verfahrensschritten zwei unterschiedliche monofunktionelle Alkohole der Formeln R¹-OH (IV) und R²-OH (V), worin R¹ und R² die in Anspruch 1 angegebene Bedeutung haben, bei einer Temperatur von 50 bis 250°C und gegebenenfalls bei einem Druck von 2 mbar bis 4 bar und gegebenenfalls in Gegenwart eines Katalysators mit Bernsteinsäure umsetzt und das gebildete Reaktionswasser durch geeignete Maßnahmen, beispielsweise Destillation, aus der Mischung entfernt.

10. Verwendung einer Estermischung oder eines Bernsteinsäureesters gemäß wenigstens einem der Ansprüche 1 bis 8 als Weichmacher für Kunststoffe und Kunststoffkomponenten.

11. Verwendung einer Estermischung oder eines Bernsteinsäureesters gemäß wenigstens einem der Ansprüche 1 bis 8 als Verarbeitungshilfsmittel und/oder Weichmacher in Klebstoffen, Klebstoffkomponenten, Klebdichtstoffen, Klebdichtstoffkomponenten, Dichtungsmassen, Dichtungsmassenkomponenten, oder Beschichtungsmassen, in Farben, Tinten oder Lacken, in Plastisolen.

12. Weichmacherzubereitung enthaltend eine Estermischung oder einen Bernsteinsäureester gemäß wenigstens einem der Ansprüche 1 bis 8.

13. Verfahren zu Herstellung von weichgemachten Kunststoffen, **dadurch gekennzeichnet, dass** man in einem ersten Schritt PVC bei 10 bis 50 °C mit einer Weichmacherzubereitung gemäß Anspruch 12 und gegebenenfalls weiteren Hilf- und Zusatzstoffen vermischt, wobei auf 100 Teile Kunststoff 10 bis 200 Teile der Weichmacherzubereitung eingesetzt werden und in einem zweiten Schritte das so hergestellte Plastisol in Form gebracht und bei Temperaturen von 140 bis 200°C zum Endartikel verarbeitet wird.

14. Plastisol enthaltend mindestens einen Kunststoff und eine Weichmacherzubereitung gemäß Anspruch 12.

## Claims

1. Mixture of succinic esters comprising compounds of the formulae
R¹-OC(O)-CH₂-CH₂-C(O)O-R¹ (I)
R¹-OC(O)-CH₂-CH₂-C(O)O-R² (II),
and
R²-OC(O)-CH₂-CH₂-C(O)O-R² (III),
in which
R¹ is a straight-chain or branched alkyl moiety, and
R² is an optionally substituted benzyl moiety,
**characterized in that** the amount of compound of the formula (I) is from 10 to 50% by weight, the amount of compound of the formula (II) is from 25 to 75% by weight and the amount of compound of the formula (III) is from 10 to 50% by weight, based on 100 percent of the mixture.

2. Ester mixture according to Claim 1, where, in the formulae (I), (II), and (III)
R¹ is a straight-chain or branched alkyl moiety having from 1 to 12 carbon atoms, and
R² is an optionally C₁-C₄-alkyl- or halogen-substituted benzyl moiety.

3. Ester mixture according to Claims 1 and 2, **characterized in that**, in the formulae (I), (II), and (III), R¹ is a straight-chain or branched alkyl moiety having from 8 to 12 carbon atoms, and R² is a benzyl moiety.

4. Ester mixture according to at least one of Claims 1 to 3, **characterized in that** the amount of compound of the formula (I) is from 15 to 35% by weight, and very particularly preferably from 20 to 30% by weight, the amount of compound of the formula (II) is from 35 to 65% by weight, and very particularly preferably from 40 to 60% by weight, and the amount of compound of the formula (III) is from 15 to 35% by weight, and very particularly preferably from 20 to 30% by weight, based in each case on 100 percent of the mixture.

5. Mixture of succinic esters comprising compounds of the formulae
R¹-OC(O)-CH₂-CH₂-C(O)O-R¹ (I)
and
R²-OC(O)-CH₂-CH₂-C(O)O-R² (III)
where the general and preferred definitions of the moieties R¹ and R² are those stated in Claims 1 to 3,
**characterized in that** the amount of compound of the formula (I) is from 15 to 95% by weight and the amount of compound of the formula (III) is from 85 to 5% by weight, based on 100 percent of the mixture.

6. Ester mixture according to Claim 5, **characterized in that** the amount of compound of the formula (I) is from 25 to 75% by weight, and very particularly preferably from 40 to 60% by weight, and the amount of compound of the formula (III) is from 75 to 25% by weight, and very particularly preferably from 60 to 40% by weight, based in each case on 100 percent of the mixture.

7. Alkyl succinate of the formula
R¹-OC(O)-CH₂-CH₂-C(O)O-R² (II)
in which
R¹ is isononyl, 3,5,5-trimethylhexyl, 1-decyl, isodecyl, isoundecyl or n-dodecyl, and
R² is benzyl.

8. Ester mixture according to at least one of Claims 1 to 6, **characterized in that** the succinic esters of the formulae (I), (II), and (III) derive from biomass resources, and the mixture comprises from 0.01 ppm to 1000 ppm content by mass of nitrogen atoms and from 0.01 ppm to 50 ppm content by mass of sulfur atoms, based in each case on the mixture.

9. Process for the production of an ester mixture according to Claim 1, **characterized in that** in a single process step or in two successive process steps two different monohydric alcohols of the formulae R¹-OH (IV), and R²-OH (V), in which R¹ and R² are defined as in Claim 1, are reacted at a temperature of from 50 to 250°C and optionally at a pressure of from 2 mbar to 4 bar and optionally in the presence of a catalyst with succinic acid, and the resultant water of reaction is removed from the mixture by suitable measures, such as distillation.

10. Use of an ester mixture or of a succinic ester according to at least one of Claims 1 to 8 as plasticizer for plastics and plastics components.

11. Use of an ester mixture or of a succinic ester according to at least one of Claims 1 to 8 as processing aid and/or plasticizer in adhesives, in components of adhesives, in adhesive sealants, in components of adhesive sealants, in sealing compositions, in components of sealing compositions, or in coating compositions, in paints, inks, or coating materials, or in plastisols.

12. Plasticizer preparation comprising an ester mixture or comprising a succinic ester according to at least one of Claims 1 to 8.

13. Process for production of plasticized plastics, **characterized in that** in a first step PVC is mixed at from 10 to 50°C with a plasticizer preparation according to Claim 12 and optionally with other auxiliaries and additives, where from 10 to 200 parts of the plasticizer preparation are used for every 100 parts of plastic, and in a second step the resultant plastisol is molded and, at temperatures of from 140 to 200°C, processed to give the final product.

14. Plastisol comprising at least one plastic and one plasticizer preparation according to Claim 12.

## Revendications

1. Mélange d'esters de l'acide succinique, contenant des composés des formules
R¹-OC(O)-CH₂-CH₂-C(O)O-R¹ (I)
R¹-OC(O)-CH₂-CH₂-C(O)O-R² (II),
et
R²-OC(O)-CH₂-CH₂-C(O)O-R² (III)
dans lesquelles
R¹ représente un radical alkyle linéaire ou ramifié et
R² représente un radical benzyle le cas échéant substitué,
**caractérisé en ce que** la quantité de composé de formule (I) est de 10 à 50% en poids, la quantité de composé de formule (II) est de 25 à 75% en poids et la quantité de composé de formule (III) est de 10 à 50% en poids, par rapport à 100% du mélange.

2. Mélange d'esters selon la revendication 1, où, dans les formules (I), (II) et (III)
R¹ représente un radical alkyle linéaire ou ramifié comprenant 1 à 12 atomes de carbone et
R² représente un radical benzyle le cas échéant substitué par C₁-C₄-alkyle ou halogène.

3. Mélange d'esters selon les revendications 1 et 2, **caractérisé en ce que**, dans les formules (I), (II) et (III), R¹ représente un radical alkyle linéaire ou ramifié comprenant 8 à 12 atomes de carbone et R² représente un radical benzyle.

4. Mélange d'esters selon au moins l'une quelconque des revendications 1 à 3, **caractérisé en ce que** la quantité de composé de formule (I) est de 15 à 35% en poids et de manière tout particulièrement préférée de 20 à 30% en poids, la quantité de composé de formule (II) est de 35 à 65% en poids et de manière tout particulièrement préférée de 40 à 60% en poids et la quantité de composé de formule (III) est de 15 à 35% en poids, de manière tout particulièrement préférée de 20 à 30% en poids, à chaque fois par rapport à 100% du mélange.

5. Mélange d'esters de l'acide succinique, contenant des composés des formules
R¹-OC(O)-CH₂-CH₂C(O)O-R¹ (I)
et
R²-OC(O)-CH₂-CH₂-C(O)O-R² (III),
les radicaux R¹ et R² présentant les significations générales et préférées indiquées dans les revendications 1 à 3, **caractérisé en ce que** la quantité de composé de formule (I) est de 15 à 95% en poids et la quantité de composé de formule (III) est de 85 à 5% en poids, par rapport à 100% du mélange.

6. Mélange d'esters selon la revendication 5, **caractérisé en ce que** la quantité de composé de formule (I) est de 25 à 75% en poids et de manière tout particulièrement préférée de 40 à 60% en poids et la quantité de composé de formule (III) est de 75 à 25% en poids et de manière tout particulièrement préférée de 60 à 40% en poids, à chaque fois par rapport à 100% du mélange.

7. Ester alkylique de l'acide succinique de formule
R¹-OC(O)-CH₂-CH₂-C(O)O-R² (II)
dans laquelle
R¹ représente isononyle, 3,5,5-triméthylhexyle, 1-décyle, isodécyle, iso-undécyle ou n-dodécyle, et
R² représente benzyle.

8. Mélange d'esters selon au moins l'une quelconque des revendications 1 à 6, **caractérisé en ce que** les esters de l'acide succinique des formules (I), (II) et (III) proviennent de ressources de biomasse et le mélange contient 0,01 ppm à 1000 ppm de proportion massique d'atomes d'azote et 0,01 ppm à 50 ppm de proportion massique d'atomes de soufre, à chaque fois par rapport au mélange.

9. Procédé pour la préparation d'un mélange d'esters selon la revendication 1, **caractérisé en ce qu'**on transforme, dans une étape de procédé ou dans deux étapes de procédé successives, deux alcools monofonctionnels différents des formules R¹-OH (IV) et R²-OH (V), dans lesquelles R¹ et R² présentent la signification indiquée dans la revendication 1, à une température de 50 à 250°C et le cas échéant à une pression de 2 mbars à 4 bars et le cas échéant en présence d'un catalyseur avec de l'acide succinique et on élimine du mélange, par des mesures appropriées, par exemple par distillation, l'eau de réaction formée.

10. Utilisation d'un mélange d'esters ou d'un ester de l'acide succinique selon au moins l'une quelconque des revendications 1 à 8 comme plastifiant pour des matériaux synthétiques ou des composants de matériau synthétique.

11. Utilisation d'un mélange d'esters ou d'un ester de l'acide succinique selon au moins l'une quelconque des revendications 1 à 8 comme adjuvant de mise en oeuvre et/ou plastifiant dans des adhésifs, des composants d'adhésif, des matériaux d'étanchéité adhésifs, des composants de matériau d'étanchéité adhésif, des masses de bouchage, des composants de masse de bouchage ou des masses de revêtement, dans des peintures, des encres ou des laques, dans des plastisols.

12. Composition de plastifiant contenant un mélange d'esters ou un ester de l'acide succinique selon au moins l'une quelconque des revendications 1 à 8.

13. Procédé pour la préparation de matériaux synthétiques plastifiés, **caractérisé en ce que**, dans une première étape, on mélange du PVC à 10 jusqu'à 50°C avec une composition de plastifiant selon la revendication 12 et le cas échéant d'autres adjuvants et additifs, 10 à 200 parties de la composition de plastifiant étant utilisées pour 100 parties de matériau synthétique et, dans une deuxième étape, on moule le plastisol ainsi préparé et on le transforme à des températures de 140 à 200°C en objet fini.

14. Plastisol contenant au moins un matériau synthétique et une composition de plastifiant selon la revendication 12.
